Europäisches Patentamt

European Patent Office

Office européen des brevets

(11) Veröffentlichungsnummer: **0 036 065**
**B1**

(12) **EUROPÄISCHE PATENTSCHRIFT**

(45) Veröffentlichungstag der Patentschrift: 03.04.85

(51) Int. Cl.⁴: **C 12 M 1/00**

(21) Anmeldenummer: **81100345.8**

(22) Anmeldetag: **17.01.81**

(54) **Verfahren zur Erzeugung von Biogas sowie Vorrichtung zur Durchführung des Verfahrens.**

(30) Priorität: **17.03.80 DE 3010183**

(43) Veröffentlichungstag der Anmeldung:
**23.09.81 Patentblatt 81/38**

(45) Bekanntmachung des Hinweises auf die
Patenterteilung:
**03.04.85 Patentblatt 85/14**

(84) Benannte Vertragsstaaten:
**AT BE CH DE FR GB IT LI LU NL SE**

(56) Entgegenhaltungen:
**DE-A-2 728 353**
**FR-A- 732 738**
**US-A-2 429 589**

(73) Patentinhaber: **Günter, Edmund**
**Küferweg 2**
**D-7891 Ühlingen-Birkendorf (DE)**

(72) Erfinder: **Günter, Edmund**
**Küferweg 2**
**D-7891 Ühlingen-Birkendorf (DE)**

(74) Vertreter: **Patentanwälte Dipl.-Ing. Hans Schmitt**
**Dipl.-Ing. Wolfgang Maucher**
**Dreikönigstrasse 13**
**D-7800 Freiburg i.Br. (DE)**

Courier Press, Leamington Spa, England.

EP 0 036 065 B1

## Beschreibung

Die Erfindung betrifft ein Verfahren zur Erzeugung von Biogas aus insbesondere landwirtschaftlichen, organischen Reststoffen (Fäkalien), wobei die Reststoffe in einem Gärbehälter gefüllt werden und dort ausgasen. Bekanntermaßen werden in einem Gärbehälter befindliche Reststoffe (Fäkalien) bei einer Temperatur von z. B. 35°C biologisch zersetzt. Dabei wird sogenanntes Biogas freigesetzt. Um zu vermeiden, daß sich auf der Fäkalienoberseite eine Schwimmdecke bildet, die eine Abgabe von Biogas aus den Fäkalien verhindert bzw. erschwert, ist es bereits bekannt, in diese Behälter Rührer auch zum Durchmischen der Reststoffe einzubauen. Zum gleichen Zweck ist es weiterhin bekannt, Biogas zurück in die Fäkalien zu leiten, wodurch ebenfalls eine Durchmischung sowie auch ein Aufreißen der Schwimmdecke stattfindet.

In der Praxis hat es sich gezeigt, daß die gasbildenden Bakterien auf verschiedene Einflußfaktoren vergleichsweise empfindlich reagieren, wobei neben der gewissen Empfindlichkeit auf absolute Werte von z. B. Temperatur, Säuergehalt, pH-Wert u. dgl. auch insbesondere eine Empfindlichkeit gegenüber vergleichsweise schnellen Änderungen dieser Einflußfaktoren beobachtet werden konnte. Gerade dies tritt aber in der Regel bei den bekannten Verfahren zur Biogaserzeugung beim Durchmischen oder Rühren der Fäkalien auf. Das Ausgasen des Biogases aus den Fäkalien kann zwar dabei momentan während einer Rühr- oder Mischperiode erhöht werden, jedoch tritt dann eine Phase geringerer Biogasproduktion bedingt durch die vorherige "Störung" des bakteriellen Umwandlungsprozesses ein.

Aufgabe der vorliegenden Erfindung ist es, eine Verfahren der eingang erwähnten Art zu schaffen, bei dem die vorerwähnten Einflußfaktoren, die zu einer nachteiligen Reduzierung des bakteriellen Umwandlungsprozesses führen, vermieden werden. Gleichzeitig soll dabei jedoch die Biogasproduktion insgesamt verbessert werden.

Zur Lösung dieser Aufgabe wird erfindungsgemäß vorgeschlagen, daß die Reststoffe jeweils von unten her kontinuierlich vergleichsweise langsam eingebracht, im wesentlichen still, rühr- bzw. mischfrei vergärt und an der Oberfläche unter Freigabe von Biogas unter Einwirkung der Schwerkraft nach unten abgeleitet werden, wobei sie an ihrer Oberfläche mit Überdruck beaufschlagt werden.

Der notwendige Umwandlungsprozeß zur Biogasproduktion kann dabei in vorteilhafter Weise ohne die eingangs erwähnten Störfaktoren ablaufen, so daß die Stoffwechselleistung der gasbildenden Bakterien nicht nachteilig beeinflußt wird.

Die Förderung von unten nach oben der Reststoffe erfolgt dabei durch das Zuführen von frischen Reststoffen, wobei dies jedoch vergleichsweise langsam und in bestimmten Zeitabständen erfolgt. Bei dem erfindungsgemäßen Verfahren werden dabei in vorteilhafter Weise die frisch zugeführten Reststoffe, die gegebenenfalls gegenüber den bereits in Vergärung befindlichen Reststoffen eine andere Temperatur, einen anderen Säuregehalt und auch andere pH-Werte haben können, allmählich an die neuen Umgebungsbedingungen angepaßt. Die Druckbeaufschlagung verbessert die Ableitung der aufschwimmenden Reststoffe, die gemäß einer Weiterbildung nochmals gären und Gas abgeben können und durch den Überdruck einer weiteren Gärstation zugeführt werden können.

Ein Verfahren von eigener schutzwürdiger Bedeutung, welches gleichzeitig eine Weiterbildung der Erfindung darstellt, kann darin bestehen, daß die Ausgärung schrittweise in mehreren nacheinander von den Reststoffen durchlaufenen Stationen erfolgt. Wie vorstehend bereits erwähnt, ist dabei der Überdruck, dem die zu vergärende Masse jeweils ausgesetzt ist, besonders vorteilhaft und hilfreich, weil so die Beförderung der mehrfach zu vergärenden Reststoffe vereinfacht wird.

Eine weitere besonders zweckmäßige Ausgestaltung der Erfindung, die ihrerseits wiederum eigene schutzwürdige Bedeutung haben kann, kann dabei noch darin bestehen, daß zuerst in einer ersten Station die bei der Ausgärung entstehenden flüchtigen Säuren abgeschieden und abgeleitet und in wenigstens einer weiteren Station Methan erzeugt und abgezogen werden. Es ist nämlich bekannt, daß bei der biologischen Ausfaulung oder Vergärung in einer ersten Stufe organische Stoffe von verschiedenen Bakteriengruppen zu flüchtigen Säuren abgebaut werden. Diese verschlechtern den Brennwert des gesamten Gasgemisches, da sie unbrennbar sind. Durch die vorbeschriebene Ausgestaltung der Erfindung können nun diese flüchtigen Säuren abgetrennt und ausgeschieden werden, so daß das eigentliche Gas einen höheren Brennwert erhält.

Zusätzlich besteht auch die Aufgabe, eine Vorrichtung zur Durchführung des erfindungsgemäßen Verfahrens zu schaffen. Diese Vorrichtung weist eine Zuführung für insbesondere landwirtschaftliche, organische Reststoffe (Fäkalien) zu wenigstens einem Gärbehälter auf und weiterhin einen Gassammelbehälter sowie ein Schlammgrube od. dgl. Die erfindungsgemäße Vorrichtung ist dadurch gekennzeichnet, daß eine Zuführöffnung für Reststoffe im unteren Bereich des Gärbehälters vorgesehen ist und daß im Gärbehälter wenigstens ein Fallrohr angeordnet ist, dessen Eintrittsöffnung sich im oberen Bereich des Gärbehälters befindet, wobei das Innere des Gärbehälters unter Überdruck steht.

Die Zufuhr von frischen Reststoffen erfolgt dabei vergleichsweise langsam, so daß eine nachteilige Beeinflussung der sich bereits im Gärbehälter befindlichen Reststoffe vermieden wird. Nach der Zufuhr im unteren Bereich des Gärbehälters werden diese allmählich unter Zunahme der Gär-Aktivität nach oben gefördert. Die eigentliche Gasfreigabe aus den Fäkalien erfolgt im wesentlichen beim Eintritt in das Fallrohr. Dabei ist von wesentlicher Bedeutung, daß hier das Problem der sogenannten Schwimmdecke, die einen oberen Abschluß der Fäkalienfüllung bildet, nicht auftritt, da ein Ausgasen aus dem Gesamtvolumen der Reststoffe hier weitgehend nicht vorgesehen ist. Vorteilhaft ist ferner, daß das Biogas innerhalb des Gärbehälters unter Überdruck steht. Dadurch besteht die Möglichkeit, auch Misch-Reststoffe, die unter anderem aufschwimmende Leichstoffe enthalten, störungsfrei zu verarbeiten. Durch die Druckbeaufschlagung werden nämlich auch diese Leichstoffe durch das Fallrohr abtransportiert, so daß an dessen oberem Ende keine Verstopfung auftritt.

Bevorzugt können mehrere in Durchlaufrichtung hintereinander beschaltete Gärbehälter vorgesehen sein, die bodenseitig Eintrittsöffnungen aufweisen, wobei die zusätzlichen Gärbehälter jeweils mit dem unteren Ende des Fallrohres eines in Durchlaufrichtung davor befindlichen Gärbehälters verbunden sind. Dadurch ist die Möglichkeit gegeben, die gesamte Anlage zur Biogaserzeugung der anfallenden Menge von Reststoffen im Laufe der Zeit anzupassen, wobei die Behälteranzahl, z. B. bei einem sich vergrößernden landwirtschaftlichen Betrieb, entsprechend angepaßt werden kann. Da die Gesamtverweildauer innerhalb der Anlage in Abhängigkeit der verwendeten Reststoffe im wesentlichen festliegt, würde sich bei einer Erhöhung der Anzahl der Gärbehälter auch die Durchlaufgeschwindigkeit entsprechend erhöhen.

Vor allem die vorbeschriebenen Maßnahmen erlauben aurßerdem, daß ein erster Gärbehälter einen vorzugsweise über ein Gegendruckventil od. dgl. führenden eigenen oder von einem Gassammelbehälter abkuppelbaren Auslaß für die in ihm erzeugten flüchtigen Säuren aufweist. Es kann also praktisch der erste Gärbehälter ausgenutzt werden, diese normalerweise den Brennwert verschlechternden Stoffe und Gase abzutrennen. Dies ist ein ganz besonderer Vorteil der Anordnung mit mehreren Gärbehältern hintereinander.

Vorzugsweise ist zur Beschickung des in Durchlaufrichtung ersten Gärbehälters eine Pumpe vorgesehen. Dadurch erfolgt nur von Zeit zu Zeit in der Regel in kürzeren Abständen ein Nachfüllen von Fäkalien in vergleichsweise geringer Menge, so daß auch dadurch die bereits in Gärung befindlichen Fäkalien praktisch nicht nachteilig beeinflußt werden. Dazu trägt auch eine Weiterbildung· der Erfindung bei, wonach die Pumpe thermostatisch in Abhängigkeit von der Temperatur der zu fördernden Fäkalien gesteuert ist, wobei der Pumpe vorzugsweise ein Heizregister od. dgl. vorgeschaltet ist. Dadurch werden nur Reststoffe mit in etwa an die bereits in Gärung befindlichen Fäkalien angepaßter Temperatur eingefördert.

Eine erfindungswesentliche Ausgestaltung sieht vor, daß die Höhen der Fallrohr-Eintrittsöffnungen und zweckmäßigerweise auch die Gärbehälter-Höhen in Durchlaufrichtung abnehmen. Dadurch ist ein hydrostatisches Gefälle geschaffen, wodurch eine Förderung von Behälter zu Behälter gegen- bzw. unterstützt ist.

Zusätzlich oder stattdessen kann vorgesehen sein, daß die Gasdruckbeaufschlagung innerhalb der Gärbehälter in Durchlaufrichtung von Behälter zu Behälter abnimmt, beispielsweise um jeweils 0,1 bis 0,4 bar, vorzugsweise 0,2 bar. Dadurch ist von Behälter zu Behälter eine Gasdruckdifferenz geschaffen, die ein Durchfördern insbesondere von Schwimm- bzw. Leichtstoffen durch die Fallrohre begünstigt.

Zweckmäßigerweise ist das Fallrohr innerhalb eines Gärbehälters etwa zentral angeordnet. Dadurch ist ein günstiges Abführen der Fäkalien in. das Fallrohr möglich. Insbesondere mit dieser zentralen Anordnung des Fallrohres innerhalb des Gärbehälters ist es nach einer Weiterbildung der Erfindung vorteilhaft, wenn der obere Gärbehälterabschluß kegelförmig nach oben zulaufend ausgebildet ist. Dadurch wird die sich auf der Oberseite der Fäkalien bildende Schwimmdecke beim Hochsteigen der Füllung auf eine vergleichsweise kleine Fläche zusammengeschoben, wobei diese Fläche dann auch etwa dem Querschnitt der Fallrohreintrittsöffnung entsprechen kann. Dadurch wird praktisch die gesamte Schwimmdecke beim Einfließen in das Fallrohr zerstört. Dadurch ist eine besonders intensive Biogas abgabe aus den Fäkalien möglich.

Um die bereits vorerwähnten unterschiedlichen Gasdruckbeaufschlagungen innerhalb der Gärbehälter zu erreichen, ist zweckmäßigerweise vorgesehen, daß sich innerhalb von den Gasentnahmeleitungen Überdruckventile befinden, die auf den jeweiligen Wert der Druckbeaufschlagung eingestellt sind.· Es wird dadurch bei dem jeweiligen Gärbehälter nur dann Biogas in einen Gasvorratsbehälter abgeleitet, wenn der Gasdruck sich über den am Überdruckventil eingestellten Wert aufgebaut hat. Durch die unterschiedliche abgestufte Einstellung der Überdruckventile bei mehreren Behältern können die jeweils gewünschten Druckdifferenzen von Behälter zu Behälter eingestellt werden.

Ein wesentlicher Vorteil der Erfindung liegt noch darin, daß die Vorrichtung im wesentlichen als geschlossenes System ausgebildet ist. Neben einer erhöhten Betriebssicherheit ist dadurch auch eine umweltfreundliche Verarbeitung der Fäkalien ermöglicht.

Zusätzliche Ausgestaltungen der Erfindung sind in den weiteren Unteransprüchen aufgeführt. Nachstehend ist die Erfindung mit ihren wesentlichen Einzelheiten anhand der Zeichnung noch näher erläutert. Es zeigt in schematischer Darstellung:

Fig. 1 eine Gesamt-Vorrichtung zur Biogaserzeugung,

Fig. 2 eine abgewandelte Ausführungsform insbesondere der Anordnung von Gärbehältern und

Fig. 3 eine der Fig. 1 entsprechende Vorrichtung, bei welcher der Auslaß des ersten Gärbehälters von einem Gassammelbehälter abgekuppelt oder von vornherein unabhängig ist.

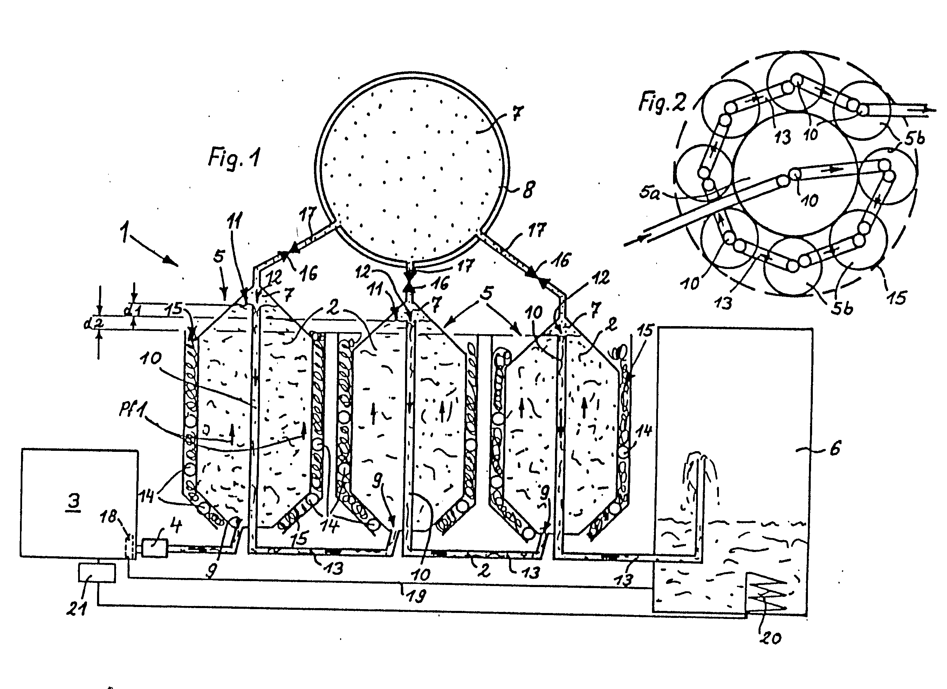

Eine im ganzen mit 1 bezeichnete Vorrichtung (Fig. 1) dient zur Erzeugung von Biogas aus insbesondere landwirtschlaftlichen, organischen Reststoffen, z. B. Fäkalien 2. Mit dieser Vorrichtung können sowohl Flüssigmiste (Gülle) als auch Misch-Reststoffe mit festeren Bestandteilen verarbeitet werden. Die Vorrichtung 1 weist als wesentliche Bestandteile in Durchlaufrichtung der Fäkalien 2 gesehen, folgende Anlagenteile auf; Eine Sammelgrube 3, eine Förderpumpe 4, einen oder mehrere Gärbehälter 5 sowie eine Schlammgrube 6. Außerdem ist für das erzeugte Biogas 7, das in Fig. 1 punktiert angedeutet ist, ein Gasvorratsbehälter 8 vorgesehen.

Die Gärbehälter 5 weisen in ihrem unteren Bereich jeweils eine Zuführöffnung 9 für Reststoffe 2 auf. Bei dem in Durchlaufrichtung ersten Gärbehälter 5, der in Fig. 1 links angeordnet ist, werden die Fäkalien 2 mittels der Förderpumpe 4 bei der Zuführöffnung 9 eingefördert. Die Fäkalien wandern dann allmählich gemäß den Pfeilen Pf 1 nach oben, bis sie mit ihrer Oberseite beim Eintrittsbereich eines innerhalb des Gärbehälters 5 angeordneten Fallrohres 10 angelangt sind. Die Fäkalien 2 gelangen dann durch das Fallrohr 10 nach unten außerhalb des Gärbehälters 5. Beim Einlaufen der Fäkalien 2 in die Eintrittsöffnung 12 des Fallrohres 10 wird eine sich gegebenenfalls ausgebildete Schwimmdecke 11 an der Oberfläche der Fäkalien 2 aufgerissen, so daß das sich bereits gebildete Biogas aus den Fäkalien 2 nach oben entweichen kann. Fig. 1 läßt gut erkennen, daß der obere Gärbehälterabschluß kegelförmig nach oben zulaufend ausgebildet ist. Dadurch wird die Schwimmdecke beim Aufsteigen der Fäkalien innerhalb des Behälters 5 zusammengeschoben und so zumindest zu einem großen Teil dem Fallrohr 10 zugeführt. Bei entsprechender Dimensionierung der Kegelform des oberen Gärbehälterabschlusses sowie auch des lichten Querschnittes des Fallrohres 10 kann dadurch praktisch die gesamte Fäkalienoberseite in das Fallrohr 10 eingeleitet werden. Das Fallrohr 10 tritt vorzugsweise an der tiefsten Stelle des Gärbehälters 5 aus, wobei entgegen der Darstellung in Fig. 1 im unteren Bereich des Fallrohres auch zur besseren Weiterleitung der Fäkalien ein gerundeter oder schräger Übergang zu der sich anschließenden Verbindungsleitung 13 vorgesehen sein kann.

Da der biochemische Umwandlungsprozeß bei der Erzeugung von Biogas 7 nur ab einer bestimmten Temperatur wirtschaftlich abläuft, sind die Gärbehälter 5 beheizt, vorzugsweise mittels einer Außenmantelheizung, von der in Fig. 1 die geschnitten dargestellten Mantelrohre 14 erkennbar sind. Zur Vermeidung von Wärmeverlusten sind die Gärbehälter 5 zweckmäßigerweise nach außen hin durch eine Isloerschicht 15 abgeschlossen. Die Ummantelung der Gärbehälter 5 kann dabei auch also kombinierte Isolier- und Stützummantelung ausgebildet sein. Eine zweckmäßige Ausführungsform sieht dabei vor, daß die Gärbehälter 5 aus vergleichsweise dünnwandigem Polyester- od. dgl. Kunststoff bestehen sowie eine äußere Stützummantelung z. B. aus Beton aufweisen. Erwähnt sei noch, daß neben der Mantelheizung auch eine Tauchheizung vorgesehen sein kann.

Im vorliegenden Ausführungsbeispiel gemäß Fig. 1 sind drei Gärbehälter 5 vorgesehen. Dabei sind die weiteren Gärbehälter 5 jeweils mit ihren Zuführöffnungen 9 über die Verbindungsleitungen 13 jeweils mit den unteren Enden des Fallrohres eines in Förderrichtung gesehen davor befindlichen Gärbehälters 5 verbunden. Die Anordnung, Anzahl sowie auch Größe der Gärbehälter richtet sich unter anderem nach der Menge der anfallenden Fäkalien 2, nach den örtlich-räumlichen Gegebenheiten sowie auch nach den gegebenenfalls vorgesehenen Erweiterungsmöglichkeiten. Beispielsweise besteht die Möglichkeit, zunächst nur einen einzigen Gärbehälter 5 vorzusehen und dann später bei größeren anfallenden Mengen von Fäkalien 2 weitere Behälter 5 anzuschließen.

Fig. 2 zeigt eine abgewandelte Ausführungsform der Anordnung von mehreren Gärbehältern 5, wobei hier ein erster Gärbehälter 5 a zentral angeordnet ist und mehrere kleine Gärbehälter 5 b außen herum um diesen ersten Gärbehälter 5 a angeordnet sind. Um diese gesamte Anordnung kann dann eine sie umschließende Isolierung 15 vorgesehen sein. Diese Anordnung hat neben einer kompakten Bauform noch den Vorteil einer günstigen Wärmeenergiehaltung.

Erwähnt sei noch, daß die Behälter 5 b gegebenenfalls auch gleich groß oder größer als der erste Gärbahälter 5 a sein können. Bei der Anordnung von mehreren in Reihe geschalteten Gärbehältern 5 bzw. 5 a, 5 b ist es vorgesehen, daß die Höhen der Fallrohr-Eintrittsöffnungen 12 und zweckmäßigerweise auch die Gärbehälter-Höhen in Durchlaufrichtung abnehmen. Dadurch ist ein hydrostatisches Gefälle geschaffen, welches die Durchförderung der Fäkalien 2 durch die in Reihe geschalteten Behälter zumindest unterstützt. Die Höhendifferenz d 1, d 2 usw. der Eintrittsöffnungen 12 kann dabei in Abhängigkeit von der Konsistenz der Reststoffe sowie dem lichten Querschnitt der Fallrohre 10 vorgesehen sein. Beispielsweise kann die Höhendifferenz der Eintrittsöffnungen 12 von einem Behälter zum nächsten etwa 0,2 m bis 1 m, vorzugsweise 0,5 m betragen. Um einen störungsfreien Betrieb bezüglich der Durchförderung der Fäkalien 2 durch die Gärbehälter 5 sicherzustellen, insbesondere auch um in den Fäkalien 2 befindliche Schwimmstoffe (Leichtstoffe) weiterzutransportieren, steht das Biogas 7 innerhalb des jeweiligen Gärbehälters 5 unter Überdruck. Dabei ist die Gasdruckbeaufschlagung innerhalb der Gärbehälter 5 so vorgesehen, daß sie in Durchlaufrichtung von Behälter zu Behälter abnimmt, beispielsweise um jeweils 0,1 bis 0,4 bar, vorzugsweise um 0,2 bar. Die unterschiedliche Gasdruckbeaufschlagung kann man an Überdruckventilen 16 einstellen, die sich innerhalb der Gasentnahmeleitungen 17 zwischen den Gärbehältern 5 und dem Gasvorratsbehälter 8 befinden.

Die Gas-Druckbeaufschlagung innerhalb der Gärbehälter 5 ist ebenfalls wie das hydrostatische Gefälle in Abhängigkeit von der Konsistenz der Reststoffe sowie dem lichten Querschnitt der entsprechenden Fallrohre vorgesehen und beträgt beispielsweise etwa 0,2 bar bis 1,5 bar.

Da die Gesamtverweildauer der Fäkalien 2 innerhalb der Vorrichtung 1 beispielsweise 20 bis 30 Tage beträgt, ist auch die Fördergeschwindigkeit der Fäkalien 2 innerhalb von den Gärbehältern 5 vergleichsweise gering. Das Nachfördern von frischen Fäkalien 2 aus der Sammelgrube 3 erfolgt mittels der Förderpumpe 4, die nur in bestimmten Zeitabständen arbeitet. Innerhalb von den Gärbehältern 5 findet keine Durchmischung z. B. mittels Rührwerken oder ein Durchgasen mittels Biogas statt. Es ist somit eine weitgehend stille Vergärung gegeben, durch die sich eine besonders günstige Bakterienaktivität zur Biogaserzeugung ergibt. Es wird bei diesem Verfahren insbesondere auch vermieden, daß frisch zugeführte Fäkalien 2 mit gegebenenfalls unterschiedlicher Temperatur und auch anderen Eigenschaften bzw. Zusammensetzungen gegenüber den dem bereits in dem Gärbehälter 5 befindlichen Fäkalien, mit diesen vermischt werden. Es hat sich in der Praxis herausgestellt, daß durch eine solche Vermischung die Aktivität der Bakterien verringert wird. Dagegen ist es bei dem erfindungsgemäßen Verfahren vorgesehen, daß bei dem langsamen Weiterfördern der Fäkalien 2 ohne Durchmischung diese Nachteile nicht auftreten. Zur Anpassung der Temperatur der frischen Fäkalien an die bereits im Behälter 5 befindlichen Fäkalien ist der Förderpumpe 4 ein Heizregister 18 vorgeschaltet, mit dem die frischen Fäkalien 2 auf einen Temperaturwert aufgeheizt werden können, der etwa auch dem zumindest im unteren Bereich des ersten Gärbehälters 5 befindlichen Fäkalien entspricht. Zweckmäßigerweise ist dabei die Pumpe 4 thermostatisch in Abhängigkeit von den zu fördernden Fäkalien 2 gesteuert.

Fig. 1 läßt noch gut erkennen, daß das jeweils untere Ende der Gärbehälter 5 trichterförmig ausgebildet ist. Absinkende Schwerstoffe aus den Fäkalien 2 können dadurch zentral z. B. bei einer hier nicht dargestellten Inspektionsöffnung der Gärbehälter 5 oder durch Absaugen entfernt werden, so daß die Reinigung der Behälter erheblich vereinfacht ist. Eventuell kann das Absaugen mit der Pumpe 4 geschehen, wenn eine entsprechende Verbindung zu deren Saugseite bzw. der Grube 4 und entsprechende Ventile bzw. Steuerschieber vorgesehen sind. Gegebenenfalls kann es noch vorgesehen sein, daß die Eintrittsöffnung 12 der Fallrohre 10 trichterförmig ausgebildet sind, um das Ableiten der Fäkalien 2 an deren Oberseite zu verbessern. Es sei noch darauf hingewiesen, daß die Abmessungen des im Kopfraum der Gärbehälter 5 befindlichen Gasräume, die Kegelform des oberen Abschlusses und auch der Querschnitt der Fallrohre 10 im oberen Bereich aufeinander abgestimmt sein können, um ein bestmögliches Abführen der Fäkalien 2 einerseits und andererseits insbesondere auch um ein günstiges Ausgasen von Biogas in diesem oberen Endbereich zu ermöglichen.

Die Fallrohre 10 können einen lichten Querschnitt von ca. 20 cm bis 40 cm, vorzugsweise etwa 30 cm haben. Die Gärbehälter 5 weisen in der Regel einen lichten Innendurchmesser von etwa 1 bis 10 m, vorzugsweise etwa 3 m sowie eine Höhe von ca. 3 bis 15 m auf.

Ein wesentlicher Vorteil der erfindungsgemäßen Biogas-Anlage besteht auch darin, daß sie als geschlossenes System ausgebildet ist, was insbesondere im Hinblick auf das verarbeitete Material vorteilhaft ist. Insbesondere kann dabei auch eine Geruchsemission sowie auch der Kontakt von Fäkalien nach außen hin und damit auch die Gefahr der Übertragung von Krankheitserregern vermieden werden. Außerdem ist die Vorrichtung 1 weitgehend wartungsfrei und langlebig, da bewegliche Teile weitgehend vermieden werden.

Die Biogas-Vorrichtung 1 kann im Kreislaufverfahren arbeiten, indem abgeleitete Fäkalien wieder zugeführt werden. Der Kreislauf kann dabei so lange beibehalten werden, bis eine weitgehende Ausgasung der Fäkalien erfolgt ist. Andererseits besteht aber auch die Möglichkeit, daß die Ausgärung von Biogas im Durchgangsverfahren erfolgt, indem die weitgehend ausgegasten Reststoffe abgeleitet werden. Dies ist in Fig. 1 dargestellt. Bei der Inbetriebnahme einer solchen Biogas-Vorrichtung 1 wird in der Regel zunächst im Kreislaufverfahren gearbeitet, bis alle Behälter gefüllt sind und die Fäkalien auf die gewünschte Temperatur aufgeheizt sind. Erst dann wird auf Durchlauf umgeschaltet. Der Kreislauf kann beim Anfahren z. B. zunächst über 10 bis 15 Tage, bis ausreichend Gas produziert wird, aufrecht erhalten bleiben. In Fig. 1 erkennt man noch eine Verbindungsleitung 19, mittels der eine geringe Menge von ausgegastem Fäkalienschlamm in die Sammelgrube 3 zu den frischen Fäkalien 2 zurückgefördert wird, um diese mit Faulbakterien zu "impfen".

Außerdem erkennt man in Fig. 1 noch eine Wärmerückgewinungseinrichtung mit einem Wärmetauscher 20, mittels dem die Restwärme aus der Schlammgrube 6 über eine Wärmepumpe 21 den frischen Fäkalien in der Sammelgrube 3 zugeführt werden kann. Dadurch ist eine günstige Energieausnützung gegeben.

Neben der bereits vorerwähnten schubweisen Zuführung von frischen Fäkalien mittels der Pumpe 4 kann auch eine kontinuierliche Beschickung des ersten Gärbehälters 5 mit frischen Fäkalien vorgesehen sein. Dabei ist eine vergleichsweise geringe Fördergeschwindigkeit vorgesehen, so daß die frischen Fäkalien mit den bereits im Gärbehälter 5 befindlichen Fäkalien praktisch nicht vermischt werden. Bei dieser kontinuierlichen Zuführung bei vergleichsweise geringer Fördergeschwindigkeit ist es vorteilhaft, wenn die Sammelgrube 3 etwas erhöht angeordnet ist, so daß die Förderpumpe 4 bezüglich ihrer Saugleistung nur gering beansprucht ist. Dies ist insbesondere bei der hier vorgesehenen sehr langsamen Förderung vorteilhaft, da die üblicherweise verwendeten Schneckenpumpen od. dgl. bei dieser langsamen Fördergeschwindigkeit nur eine geringe Saugleistung aufweisen. Durch die etwas erhöhte Anordnung der Sammelgrube 3 tritt dies jedoch dann nicht nachteilig in Erscheinung.

Erwähnt sei noch, daß die erfindungsgemäße Vorrichtung auch in Kläranlagen verwendbar ist, wobei insbesondere Dick- bzw. Feststoffe (breiartig) verarbeitet werden können. Dabei könnten dann die anfallenden Fäkalien durch die biochemische Umsetzung von Schadstoffen weitgehend befreit werden und gleichzeitig könnte mit dem Kläreffekt noch Biogas erzeugt werden, das entweder innerhalb der Kläranlage Verwendung finden könnte oder aber als verwendbares Nebenprodukt anderweitig zur Verfügung steht. Die erfindungsgemäße Vorrichtung läßt sich somit überall dort vorteilhaft einsetzen, wo Fäkalien anfallen. Die Verweildauer innerhalb von der erfindungsgemäßen Vorrichtung kann dabei an die Art der Zusammensetzung der Fäkalien durch unterschiedliche Fördergeschwindigkeit und auch die Anzahl der Behälter angepaßt werden.

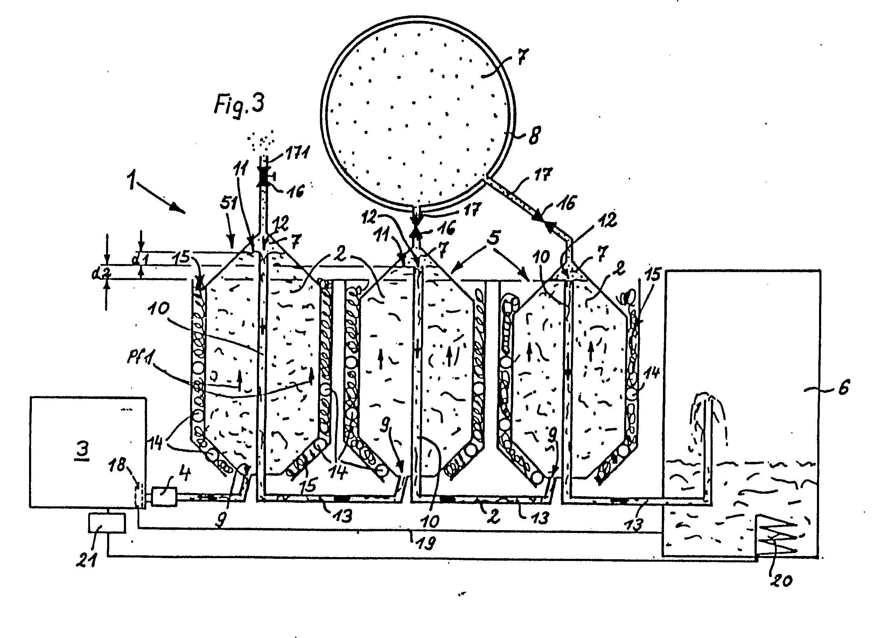

In Fig. 3 ist ein Ausführungsbeispiel dargestellt, bei welchem der erste Gärbehälter 51 einen über ein Gegendruckventil 16 führenden eigenen Auslaß 171 für in ihm erzeugte flüchtige Säuren aufweist. Bekanntermaßen werden bei Ausfaulungsprozessen zuerst von bestimmten Bakterien nicht brennbare Gase, nämlich flüchtige Säuren, freigesetzt, die auf diese Weise abgeschieden werden können. Falls dabei in diesem ersten Gärbehälter 51 auch schon Methan anfällt, kann die so entstehende, sehr minderwertige Gasmischung einfach abgefackelt werden. Auf jeden Fall ist dieser Auslaß 171 nicht mit dem Gassammelbehälter 8 verbunden. Falls bei der Ausführungsform nach Fig. 1 die Leitung 17 von diesem Gasgehälter 8 abkuppelbar ist, kann auch diese Ausführungsform in ähnlicher Weise benutzt werden.

Zweckmäßigerweise ist bei der Ausführungsform nach Fig. 3 der erste Behälter 51 so klein, daß die Verweilzeit der zu vergährenden Stoffe nur 7 Tage oder bei höherer Temperatur auch noch weniger beträgt, damit möglichst wenig Methan, welches erzeugt und benutzt werden soll, mit den flüchtigen Säuren abgeschieden wird. Der erste Behälter 52 zum Abscheiden der flüchtigen Säuren wird also in der Regel kleiner als die folgenden Behälter sein.

Die erfindungsgemäße Verfahrensweise und auch die Vorrichtung mit mehreren hintereinander geschlateten Gärbehältern hat also den erheblichen Vorteil, daß nicht nur eine gute Ausnutzung der Reststoffe biem Gewinnen brennbarer Gase möglich ist, sondern daß sogar die nicht brennbaren Bestandteile ohne wesentliche Gasverluste leicht von den brennbaren Gasen getrennt gewonnen und abgeführt werden können. Da auf diese Weise also ein wesentlich reineres Methan in den folgenden Gärbehältern 5 gewonnen werden kann, ist dieses auch höherwertig und hat bessere Brenneigenschaften.

Die vorbeschriebenen Merkmale und Maßnahmen können dabei teils einzeln, teils in Kombination Verwendung finden und es sei erwähnt, daß alle in der Beschreibung, den Ansprüche und der Zeichnung dargestellten Merkmale und Konstruktionsdetails sowohl einzeln als auch in beliebiger Kombination miteinander wesentliche Bedeutung haben.

**Patentansprüche**

1. Verfahren zur Erzeugung von Biogas aus insbesondere landwirtschaftlichen, organischen Reststoffen (Fäkalien), wobei die Reststoffe in einen Gärbehälter unter der Oberfläche von dessen Füllung eingebracht werden und dort ausgasen, dadurch gekennzeichnet, daß die Reststoffe (2) jeweils von unten her kontinuierlich vergleichsweise langsam eingebracht, im wesentlichen still, rühr- bzw. mischfrei vergärt und an der Oberfläche unter Freigabe von Biogas unter Einwirkung der Schwerkraft nach unten abgeleitet werden, wobei sie an ihrer Oberfläche mit Überdruck beaufschlagt werden.

2. Verfahren nach Anspruch 1, dadurch gekennzeichnet, daß der Überdruck durch das entstehende Gas selbst gebildet wird.

3. Verfahren nach Anspruch 1 oder 2, dadurch gekennzeichnet, daß die Ausgärung von Biogas (7) im Kreislaufverfahren erfolgt, indem abgeleitete Fäkalien (2) wieder zugeführt werden.

4. Verfahren nach einem der Ansprüche 1 bis 3, dadurch gekennzeichnet, daß die Ausgärung von Biogas (7) im Durchgangsverfahren erfolgt, indem die weitgehend ausgegasten Reststoffe (2) abgeleitet werden.

5. Verfahren nach einem der Ansprüche 1 bis 4, dadurch gekennzeichnet, daß die Ausgärung schrittweise in mehreren nacheinander von den Reststoffen (2) durchlaufenen Stationen erfolgt.

6. Verfahren nach einem der Ansprüche 1 bis 5, dadurch gekennzeichnet, daß zuerst in einer ersten Station die bei der Ausgärung entstehenden flüchtigen Säuren abgeschieden und abgeleitet und an wenigstens einer weiteren Station Methan erzeugt und abgezogen werden.

7. Vorrichtung zur Erzeugung von Biogas mit einer Zuführung für insbesondere landwirtschaftliche, organische Reststoffe (Fäkalien) zu wenigstens einem Gärbehälter, mit einem Gassammelbehälter sowie einer Schlammgrube od. dgl., zur Durchführung des Verfahrens nach einem der Ansprüche 1 bis 6, dadurch gekennzeichnet, daß eine Zuführöffnung (9) für Reststoffe (2) im unteren Bereich des Gärbehälters (5) vorgesehen ist und daß im Gärbehälter (5) wenigstens ein Fallrohr (10) angeordnet ist, dessen Eintrittsöffnung (12) sich im oberen Bereich des Gärbehälters (5) befindet, so daß die aufschwimmenden Reststoffe eintreten können, wobei das Innere des Gärbehälters (5) unter Überdruck steht.

8. Vorrichtung nach Anspruch 6 oder 7, dadurch gekennzeichnet, daß sich das Fallrohr (10) im wesentlichen innerhalb des Gärbehälters (5) befindet, insbesondere zentral angeordnet ist und vorzugsweise an dessen tiefster Stelle austritt.

9. Vorrichtung nach einem der Ansprüche 7 oder 8, dadurch gekennzeichnet, daß mehrere in Durchlaufrichtung hintereinander geschaltete Gärbehälter (5) vorgesehen sind, die bodenseitig Eintrittsöffnungen (9) aufweisen, wobei die zusätzlichen Gärbehälter (5) jeweils mit dem unteren Ende des Fallrohres (10) eines in Durchlaufrichtung davor befindlichen Gärbehälters (5) verbunden sind.

10. Vorrichtung nach einem der Ansprüche 7 bis 9, dadurch gekennzeichnet, daß ein erster Gärbehälter (51) einen vorzugsweise über ein Gegendruckventil (16) führenden eigenen oder von einem Gassammelbehälter (8) abkuppelbaren Auslaß (171) für die in ihm erzeugten flüchtigen Säuren aufweist.

11. Vorrichtung nach einem der Ansprüche 7 bis 10, dadurch gekennzeichnet, daß zur Beschickung des in Durchlaufrichtung ersten Gärbehälters (5) eine Pumpe (4) vorgesehen ist, die vorzugsweise zumindest im Bodenbereich einer Sammelgrube (3) für die Reststoffe angeordnet ist.

12. Vorrichtung nach einem der Ansprüche 7 bis 11, dadurch gekennzeichnet, daß die Höhen der Fallrohr-Eintrittsöffnungen (12) und zweckmäßigerweise auch die Gärbehälter-Höhen in Durchlaufrichtung abnehmen und/oder daß die Gas-Druckbeaufschlagung innerhalb der Gärbehälter (5) in Durchlaufrichtung von Behälter zu Behälter vorzugsweise abnimmt, beispielsweise um jeweils 0,1 bar bis 0,4 bar, vorzugsweise 0,2 bar.

13. Vorrichtung nach einem der Ansprüche 7 bis 12, dadurch gekennzeichnet, daß die Durckbeaufschlagung innerhalb der Gärbehälter (5) in Abhängigkeit von der Konsistenz der Reststoffe (2) sowie dem lichten Querschnitt des Fallrohres vorgesehen ist und beispielsweise etwa 0,2 bar bis 1,5 bar beträgt und/oder daß vorzugsweise die Höhendifferenz (d 1, d 2 usw.) der Eintrittsöffnungen (12) der Fallrohre (10) von hintereinander angeordneten Gärbehältern (5) in Abhängigkeit von der Konsistenz der Reststoffe (Fäkalien) (2) sowie dem lichten Querschnitt des Fallrohres (10) vorgesehen ist und beispielsweise etwa 0,2 m bis 1 m, vorzugsweise 0,5 m beträgt und daß vorzugsweise auch die Behälterhöhen entsprechend den unterschiedlichen Fallrohrlängen angepaßt sind.

14. Vorrichtung nach einem der Ansprüche 7 bis 13, dadurch gekennzeichnet, daß der obere Gärbehälterabschluß kegelförmig nach oben zulaufend ausgebildet ist.

15. Vorrichtung nach einem oder mehreren der Ansprüche 7 bis 14, dadurch gekennzeichnet, daß das untere Ende des Gärbehälters (5) trichterförmig ausgebildet ist, insbesondere zur Erleichterung einer Reinigung und Entfernung von Ablagerungen beispielsweise durch Absaugung.

16. Vorrichtung nach einem oder mehreren der Ansprüche 7 bis 15, dadurch gekennzeichnet, daß sich innerhalb von den Gasentnahmeleitungen (17) Überdruckventile (16) befinden, die auf den jeweiligen Wert der Druckbeaufschlagung eingestellt bzw. einstellbar sind.

17. Vorrichtung nach einem der Ansprüche 7 bis 16, dadurch gekennzeichnet, daß die Gärbehälter (5) eine Polyester- od. dgl. Kunststoffinnenschicht bzw. beschichtung sowie gegebenenfalls eine Stützummantelung vorzugsweise aus Beton aufweisen und gegebenenfalls beheizt sind, vorzugsweise mittels einer Außenmantelheizung und vorzugsweise eine Isolierummantelung (15) haben.

18. Vorrichtung nach einem der Ansprüche 7 bis 17, dadurch gekennzeichnet, daß mehrere vorzugsweise kleinere Gärbehälter (5 b) um einen insbesondere größeren Gärbehälter (5 a) angeordnet sind.

19. Vorrichtung nach einem der Ansprüche 7 bis 18, dadurch gekennzeichnet, daß vor dem ersten Gärbehälter (5, 5 a) eine Sammelgrube (3) vorgesehen ist, in der sich vorzugsweise ein der Förder-Pumpe (4) vorgeschaltetes Heizregister (18) befindet, insbesondere in der Nähe des Auslasses.

20. Vorrichtung nach einem der Ansprüche 7 bis 19, dadurch gekennzeichnet, daß die Fallrohre (10) einen lichten Querschnitt von ca. 20 cm bis 40 cm, vorzugsweise etwa 30 cm haben und gegebenenfalls bei ihrer Eintrittsöffnung (12) trichterförmig ausgebildet sind.

21. Vorrichtung nach einem der Ansprüche 7 bis 20, dadurch gekennzeichnet, daß die Beschickung des ersten Gärbehälters (5) mit frischen Fäkalien im wesentlichen kontinuierlich bei vergleichsweise geringer Fördergeschwindigkeit erfolgt und daß vorzugsweise die Sammelgrube (3) gleich hoch oder gegebenenfalls höher als der zu füllende Gärbehälter (5) oder zumindest dessen Boden angeordnet ist.

22. Vorrichtung nach des Ansprüchen 7—21 dadurch gekennzeichnet, daß der erste Gärbehälter (51) zum Abscheiden flüchtiger Säuren kleiner als die folgenden Behälter (5) ist und daß der erste Behälter (51) so klein ist, daß die Verweilzeit der Reststoffe beim Vergären circa sieben Tage oder bei höherer Temperatur weniger beträgt.

**Revendications**

1. Procédé de production de biogaz en partant de déchets organiques, en particulier agricoles (matières fécales), dans lequel on amène les déchets à un récipient de fermentation, sous la surface du contenu de celui-ci, et ils y dégagent du gaz, caractérisé en ce que l'on introduit chaque fois les déchets (2) par le bas, de façon continue et relativement lente, on les fait fermenter à l'état pratiquement calme sans agitation ni brassage, et à la surface, avec libération de biogaz, ils sont évacués vers le bas sous l'action de la pesanteur, une surpression leur étant appliquée à leur surface.

2. Procédé selon la revendication 1, caractérisé en ce que la surpression est formée par le gaz même, qui est engendré.

3. Procédé selon l'une des revendications 1 ou 2, caractérisé en ce que la fermentation de biogaz (7) s'effectue avec recyclage, des matières fécales évacuées (2) étant à nouveau amenées.

4. Procédé selon l'une des revendications 1 à 3, caractérisé en ce que la fermentation de biogaz (7) s'effectue par un procédé de passage, les déchets largement dégazés (2) étant évacués.

5. Procédé selon l'une des revendications 1 à 4, caractérisé en ce que la fermentation s'effectue par étapes, en plusieurs postes parcourus successivement par les déchets (2).

6. Procédé selon l'une des revendications 1 à 5, caractérisé en ce que l'on sépare et que l'on évacue tout d'abord, en un premier poste, les acides volatils formés lors de la fermentation, et en au moins un autre poste, on fabrique du méthane et on le retire.

7. Installation pour la production de biogaz comportant une amenée de déchets organiques, en particulier agricoles (matières fécales) à au moins un récipient de fermentation, un récipient collecteur de gaz ainsi qu'une fosse à boue ou un dispositif similaire, pour la mise en oeuvre du procédé selon l'une des revendications 1 à 6, caractérisée en ce qu'une ouverture d'amenée (9) de déchets (2) est prévue dans la région inférieure du récipient de fermentation (5), et en ce que dans le récipient de fermentation (5) est disposé au moins un tube de descente (10), dont l'ouverture d'entrée (12) se trouve dans la région supérieure du récipient de fermentation (5), de sorte que les déchets, qui montent à la surface, peuvent entrer, l'intérieur du récipient de fermentation (5) étant sous dépression.

8. Installation selon l'une des revendications 6 et 7, caractérisée en ce que le tube de descente (10) se trouve essentiellement à l'intérieur du récipient de fermentation (5), en particulier qu'il est disposé au centre et qu'il sort, de préférence, en son point le plus bas.

9. Installation selon l'une des revendications 7 et 8, caractérisée en ce qu'il est prévu plusieurs récipients de fermentation (5) branchés à la suite les uns des autres dans le sens de passage et qui présentent du côté du fond des ouvertures d'entrée (9), les récipients de fermentation supplémentaires (5) étant reliés chacun à l'extrémité inférieure du tube de descente (10) d'un récipient de fermentation (5) situé avant lui dans le sens de passage.

10. Installation selon l'une des revendications 7 à 9, caractérisée en ce qu'un premier récipient de frementation (51) présente, pour les acides volatils, qui y sont engendrés, une sortie (171) spéciale ou pouvant être désaccouplée d'un récipient collecteur de gaz (8) et passant, de préférence, par une valve de contre-pression (16).

11. Installation selon l'une des revendications 7 à 10, caractérisée en ce que pour l'alimentation du premier récipient de fermentation (5) dans le sens de passage est prévue une pompe (4) qui est, de préférence, disposée au moins dans la région de fond d'une fosse collectrice (3) destinée aux déchets.

12. Installation selon l'une des revendications 7 à 11, caractérisée en ce que la hauteur des ouvertures d'entrée (12) du tube de descente et, de préférence, aussi la hauteur des récipients de fermentation diminuent dans le sens de passage et/ou en ce que l'application de pression au gaz à l'intérieur des récipients (5) diminue, de préférence, d'un récipient à l'autre dans le sens de passage, par exemple chaque fois de 0,1 à 0,4 bar, de préférence de 0,2 bar.

13. Installation selon l'une des revendications 7 à 12, caractérisée en ce que l'application de pression à l'intérieur des récipients de fermentation (5) est prévue en fonction de la consistance des déchets (2) ainsi que de la section libre du tube de descente et qu'elle est, par exemple, d'environ 0,2 à 1,5 bar, et/ou en ce que, de préférence, la différence de hauteur (d 1, d 2, etc.) des ouvertures d'entrée (12) des tubes de descente (10) de récipients de fermentation (5) disposés à la suite les uns des autres est prévue en fonction de la consistance des déchets (matières fécales) (2) ainsi que de la section libre du tube de descente (10) et est, par exemple, d'environ 0,2 à 1 m, de préférence de 0,5 m, et en ce que, de préférence, la hauteur des récipients aussi est adaptée conformément aux différentes longueurs des tubes de descente.

14. Installation selon l'une des revendications 7 et 13, caractérisée en ce que la fermeture supérieure du récipient de fermentation a une forme rétrécissant coniquement vers le haut.

15. Installation selon une ou plusieurs des revendications 7 à 14, caractérisée en ce que l'extrêmité inférieure du récipient de fermentation (5) est constituée en forme d'entonnoir, en particulier pour faciliter le nettoyage et l'élimination des dépôts, par exemple par aspiration.

16. Installation selon une ou plusieurs des revendications 7 à 15, caractérisée en ce qu'à l'intérieur des tuyaux de retrait de gaz (17) se trouvent des limiteurs de pression (16), qui sont réglés ou peuvent être réglés à la valeur de la pression à appliquer dans le cas d'espèce.

17. Installation selon l'une des revendications 7 à 16, caractérisée en ce que les récipients de fermentation (5) présentent une couche ou un revêtement intérieur de polyester ou matière synthétique similaire ainsi qu'éventuellement un revêtement de soutien, de préférence en béton, et sont éventuellement chauffés, de préférence au moyen d'un chauffage par chemise extérieure et présentent, de préférence, un revêtement isolant (15).

18. Installation selon l'une des revendications 7 à 17, caractérisée en ce que plusieurs récipients de fermentation (5b), de préférence plus petits, et un récipient de fermentation (5a), en particulier plus grand, sont disposés.

19. Installation selon l'une des revendications 7 à 18, caractérisée en ce qu'avant le premier récipient de fermentation (5, 5a) est prévue une fosse collectrice (3), dans laquelle se trouve, de préférence, un registre de chauffage (18) placé avant la pompe (4), en particulier au voisinage de la sortie.

20. Installation selon l'une des revendications 7 à 19, caractérisée en ce que les tubes de descente (10) ont une section libre d'environ 10 à 40 cm, de préférence d'environ 30 cm et qu'éventuellement, près de leur ouverture d'entrée (12), ils sont constitués en forme d'entonnoir.

21. Installation selon l'une des revendications 7 à 20, caractérisée en ce que l'alimentation du premier récipient de fermentation (5) en matières fécales fraîches s'effectue de façon pratiquement continue, à une vitesse de transport relativement réduite, et en ce que, de préférence, la fosse collectrice (3) est disposée à la même hauteur ou éventuellement plus haut que le récipient de fermentation (5) à remplir, ou du moins son fond.

22. Installation selon les revendications 7 à 21, caractérisée en ce que le premier récipient de fermentation (51) servant à séparer les acides volatils est plus petit que les récipients suivants (5), et en ce que le premier récipient (51) est si petit que le temps de séjour des déchets lors de la fermentation est d'environ sept jours, ou éventuellement inférieur si la température est plus élevée.

## Claims

1. Process for producing biogas from, in particular, agricultural organic residual matter (faeces), in which the residual matter is introduced into a fermentation container below the surface of the contents thereof and there gives off gas, characterised in that the residual matter (2) is introduced continuously and comparatively slowly from below, fermented substantially undisturbed without stirring or mixing and at the surface, with the release of biogas is discharged downwards under the effect of gravity, the surface of the residual matter being pressurised.

2. Process as claimed in claim 1, characterised in that pressurisation is created by the gas itself as it is produced.

3. Process as claimed in claim 1 or 2, characterised in that the fermentation of biogas (7) is carried out in a cyclic process in which the faeces (2) discharged are recycled.

4. Process as claimed in one of claims 1 to 3, characterised in that the fermentation of biogas (7) is carried out in a through process in which the substantially degassed residual matter (2) is discharged.

5. Process as claimed in one of claims 1 to 4, characterised in that the fermentation is carried out step by step in a plurality of stations through which the residual matter (2) passes successively.

6. Process as claimed in one of claims 1 to 5, characterised in that initially, in a first stage, the volatile acids formed during fermentation are separated off and discharged and in at least one other station methane is produced and drawn off.

7. Apparatus for producing biogas with a means for supplying, in particular, agricultural organic residual matter (faeces) to at least one fermentation chamber, with a gas collecting chamber and a slurry pit or the like, for carrying out the process as claimed in one of claims 1 to 6, characterised in that a supply opening (9) for residual matter (2) is provided in the lower part of the fermentation container (5) and in that at least one down pipe (10) is mounted in the fermentation container (5), the inlet opening (12) of the down pipe (10) being in the upper part of the fermentation container (5) so that the residual matter floating up can flow in, the interior of the fermentation container (5) being pressurised.

8. Apparatus as claimed in claim 6 or 7, characterised in that the down pipe (10) is located substantially inside the fermentation container (5), particularly centrally, and preferably discharges at the lowest point thereof.

9. Apparatus as claimed in one of claims 7 or 8, characterised in that a plurality of fermentation containers (5) are provided, connected in series in the direction of flow, which have inlet openings (9) at their bases, the additional fermentation containers (5) being each connected to the lower end of the down pipe (10) of a preceding fermentation container (5) in the direction of flow.

10. Apparatus as claimed in one of claims 7 to 9, characterised in that a first fermentation container (51) has its own outlet (171) preferably via a backpressure valve (16) or an outlet (171) which can be disconnected from a gas collecting container (8), for the volatile acids produced in said fermentation container.

11. Apparatus as claimed in one of claims 7 to 10, characterised in that a pump (4) is provided for charging the first fermentation container (5) in the direction of flow, said pump (4) preferably being arranged at least in the region of the base of a collecting pit (3) for the residual matter.

12. Apparatus as claimed in one of claims 7 to 11, characterised in that the heights of the inlet openings (12) of the down pipes and preferably also the heights of the fermentation containers decrease in the direction of flow and/or the gas pressure acting within the fermentation containers (5) preferably decreases from one container to the next, in the direction of flow for example by 0.1 bar to 0.4 bar each time, preferably by 0.2 bar.

13. Apparatus as claimed in one of claims 7 to 12, characterised in that the pressure prevailing inside the fermentation containers (5) is selected as a function of the consistency of the residual matter (2) and the internal cross section of the down pipe and is, for example, about 0.2 bar to 1.5 bar and/or preferably the difference in height (d1, d2, etc) between the inlet openings (12) of the down pipes (10) of successive fermentation containers (5) is selected as a function of the consistency of the residual matter (faeces) (2) and the internal cross section of the down pipe (10) and is, for example, about 0.2 m to 1 m, preferably 0.5 m, and preferably also the heights of the containers are adapted to the different lengths of the down pipes.

14. Apparatus as claimed in one of claims 7 to 13, characterised in that the upper closure of the fermentation container is shaped so as to con-

9

verge conically upwards.

15. Apparatus as claimed in one or more of claims 7 to 14, characterised in that the lower end of the fermentation container (5) is funnel-shaped, particularly in order to facilitate cleaning and removal of deposits, for example by suction.

16. Apparatus as claimed in one or more of claims 7 to 15, characterised in that, inside the gas removal ducts (17), there are pressure relief valves (16) which are or can be set to the particular pressure level used.

17. Apparatus as claimed in one of claims 7 to 16, characterised in that the fermentation containers (5) have a polyester or similar plastics inner lining or coating and optionally a supporting casing, preferably of concrete, and may be heated, preferably by means of an outer jacket heating system and preferably have an insulating jacket (15).

18. Apparatus as claimed in one of claims 7 to 17, characterised in that a plurality of preferably smaller fermentation containers (5b) are arranged around a, in particular, larger fermentation container (5a).

19. Apparatus as claimed in one of claims 7 to 18, characterised in that, in front of the first fermentation container (5, 5a), there is a collecting pit (3) in which there is preferably a radiator (18) arranged ahead of the delivery pump (4), particularly near the outlet.

20. Apparatus as claimed in one of claims 7 to 19, characterised in that the down pipes (10) have an internal cross section of from about 20 cm to 40 cm, preferably about 30 cm, and are, if required, funnel-shaped at their inlet opening (12).

21. Apparatus as claimed in one of claims 7 to 20, characterised in that the charging of the first fermentation container (5) with fresh faeces is effected substantially continuously at a comparatively slow delivery speed and preferably the collecting pit (3) is the same height as or, of required, higher than the fermentation container (5) which is to be filled or at least the base thereof.

22. Apparatus as claimed in claims 7 to 21, characterised in that the first fermentation container (51) for separating off volatile acids is smaller than the following containers (5) and the first container (51) is so small that the retention time for the residual matter during fermentation is about seven days, or less, in the case of a higher temperature.

Fig.1

Fig.2

Fig.3